## Europäisches Patentamt
## European Patent Office
## Office européen des brevets

(19)

(11) Veröffentlichungsnummer: **0 141 312**
**B1**

(12) ## EUROPÄISCHE PATENTSCHRIFT

(45) Veröffentlichungstag der Patentschrift:
**20.04.88**

(51) Int. Cl.⁴: **C 07 D 251/28**

(21) Anmeldenummer: **84112227.8**

(22) Anmeldetag: **11.10.84**

(54) Verfahren zur Gewinnung von festem Cyanurchlorid.

(30) Priorität: **11.10.83 DE 3336993**

(43) Veröffentlichungstag der Anmeldung:
**15.05.85 Patentblatt 85/20**

(45) Bekanntmachung des Hinweises auf die Patenterteilung:
**20.04.88 Patentblatt 88/16**

(84) Benannte Vertragsstaaten:
**BE CH DE FR GB IT LI NL**

(56) Entgegenhaltungen:
**AT - B - 231 995**
**AT - B - 366 378**
**DE - B - 2 537 673**
**DE - B - 2 843 379**
**DE - C - 1 071 709**

**Die Akte enthält technische Angaben, die nach dem Eingang der Anmeldung eingereicht wurden und die nicht in dieser Patentschrift enthalten sind.**

(73) Patentinhaber: SKW Trostberg Aktiengesellschaft,
Dr.-Albert-Frank-Strasse 32 Postfach 1150/1160,
D-8223 Trostberg (DE)

(72) Erfinder: Elischer, Stefan, Dr., Dr.-Winnacker-Strasse 4,
D-8071 Münchsmünster (DE)
Erfinder: Höbel, Hans-Günther, Birkengrund 2,
D-8301 Aiglsbach (DE)
Erfinder: Merkt, Robert, Lindenstrasse 40,
D-8071 Münchsmünster (DE)
Erfinder: Wagner, Fritz, Dr., Dr.-Winnacker-Strasse 6,
D-8071 Münchsmünster (DE)

(74) Vertreter: Weickmann, Heinrich, Dipl.-Ing. et al,
Patentanwälte Dipl.-Ing. H.Weickmann Dipl.-Phys.Dr.
K.Fincke Dipl.-Ing. F.A.Weickmann Dipl.-Chem. B. Huber
Dr.-Ing. H. Liska Dipl.-Phys.Dr. J. Prechtel
Postfach 860820, D-8000 München 86 (DE)

ACTORUM AG

## Beschreibung

Die vorliegende Erfindung betrifft ein Verfahren zur Gewinnung von festem Cyanurchlorid in feinverteilter Form.

Cyanurchlorid, welches als Zwischenprodukt für die Herstellung von Farbstoffen, Pflanzenschutzmitteln, Pharmazeutika sowie Textil- und Kautschukhilfsmittel erhebliche technische Bedeutung besitzt, fällt nach der katalytischen Trimerisierung von Chlorcyan in gasförmiger Form an, zusammen mit nicht umgesetztem Chlorcyan und Chlor. Dieses Gasgemisch wurde üblicherweise in Abscheidekammern geleitet und das Cyanurchlorid an den gekühlten Wänden abgeschieden. Nachteilig bei dieser Art der Desublimation ist, daß sich das Cyanurchlorid in Form grober Kristalle an den Wänden und Austragsvorrichtungen absetzt und somit den Wärmeübergang negativ beeinflußt. Regelmäßiges Abklopfen der Kristalle von den Wänden führt zwar zu einer kurzzeitigen Verbesserung des Wärmeüberganges, doch ist diese Methode wegen der zunehmenden mechanischen Beschädigung und der Lärmbelästigung keineswegs befriedigend, ganz abgesehen von der schlechten Qualität des auf diese Weise erhaltenen Produkts.

Gemäß der DE-AS 12 66 308 versuchte man dieses Problem dadurch zu umgehen, daß man Cyanurchlorid zusammen mit einer leicht verdampfenden inerten Kühlflüßigkeit wie z.B. Chloroform versprüht. Man erhält auf diese Weise zwar ein feinverteiltes Cyanurchlorid, doch ist die Rückgewinnung der Kühlflüßigkeit ziemlich aufwendig. Darüber hinaus kann es sehr leicht zu Verstopfungen der Düse kommen.

Schließlich werden in der DE-AS 28 43 381 und der DE-AS 28 43 382 Verfahren zur Gewinnung von flüßigem oder festem Cyanurchlorid beschrieben, nach denen man das nach der Trimerisierung von Cyanurchlorid anfallende Reaktionsgemisch in eine Apparatekombination bestehend aus Abtriebskolonne und Kondensator einleitet und durch Temperaturregelung und Zumischen von Inertgas (zur Verhinderung von Totalkondensation) am Austritt des Kondensators das Cyanurchlorid in der Kolonne teilweise kondensiert, während der gasförmige Anteil, der am Kolonnenkopf austritt, in den üblichen Abscheidekammern desublimiert wird. Nachteilig bei dieser Methode ist die aufwendige Apparatekombination, die hohe Investitions- und Betriebskosten verursacht.

Aus der AT-B-231 995 und der DE-C-1 071 709 sind Verfahren zur Reinigung von rohem Cyanurchlorid bekannt. Nach der AT-B-231 995 erfolgt die Reinigung durch fraktionierte Destillation, wobei das fraktionierte Cyanurchlorid mit Hilfe eines Transportgases aus der Kolonne in eine Abscheidevorrichtung geleitet wird; in einer Ausführungsform erfolgt die Abkühlung in der Abscheidevorrichtung mittels eines inerten Kühlgases. Nach dem Verfahren der DE-C-1 071 709 wird das durch Sublimation von Roh-Cyanurchlorid erhaltene Cyanurchlorid-Gas mit Hilfe eines inerten Transportgases aus dem Verdampfungsraum geführt, mit einem Kühlgas gemischt und das Gemisch von Kühlgas und Transportgas, das das durch Abkühlung kristallisierte Cyanurchlorid enthält, zu einem Abscheider geführt, wo es von dem Kühl- und Transportgas abgetrennt wird. Der vorliegenden Erfindung liegt daher die Aufgabe zugrunde, ein Verfahren zur Gewinnung von festem Cyanurchlorid zu entwickeln, welches die geschilderten Nachteile des Standes der Technik nicht aufweist und ohne grossen technischen Aufwand ein Cyanurchlorid bereitstellt, das hinsichtlich der Kornverteilung und Reinheit des Produktes befriedigende Ergebnisse liefert.

Diese Aufgabe wird erfindungsgemäß dadurch gelöst, daß man den Cyanurchloriddampf und kalte Luft durch schräg nach oben gerichtete Zuleitungen derart in den unteren Teil einer Abscheidekammer einleitet, daß der Neigungswinkel der Zuleitung gegenüber der Horizontalen 10 bis 80° beträgt, die Kaltluft eine Temperatur von 0 bis 60°C besitzt und man dem Cyanurchlorid die Kaltluft in einer Menge von 20 bis 150 kg pro 100 kg Cyanurchloriddampf zudosiert.

Nach diesem Verfahren wird überraschenderweise ein äußerst feinteiliges und gleichzeitig sehr reines Cyanurchlorid erhalten, wodurch sich aufwendige Apparaturen für zusätzliche Reinigungsschritte erübrigen. Gemäß der Erfindung wird der Cyanurchloriddampf, der noch zahlreiche Verunreinigungen wie z.B. Chlorcyan oder Chlor enthält, nach dem Austritt aus dem Trimerisierungsreaktor in die Abscheidekammer geleitet. Da kein partialdruckerniedrigendes Gas zugesetzt wird, muß die Leitung 001schen Reaktor und Abscheidekammer wärmeisoliert werden, um eine vorzeitige Kondensation des Cyanurchlorids zu verhindern, die zu unerwünschten Verstopfungen führen kann.

Das gasförmige Reaktionsgemisch sollte vor dem Eintritt in die Abscheidekammer zweckmäßigerweise eine Temperatur zwischen 250 und 300°C aufweisen, damit ein guter Abschreckeffekt durch die Kaltluft gewährleistet ist. Die heißen Cyanurchloriddämpfe werden durch eine schräg nach oben gerichtete Zuleitung in den unteren Teil der Abscheidekammer zusammen mit getrennt zugeführter kalter Luft eingeblasen, wobei die Kaltluft vorzugsweise eine Temperatur von 10 bis 25°C besitzt. Die Zuleitung weist gegenüber der Horizontalen einen Winkel von 10 bis 80°, vorzugsweise von 30 bis 50° auf, damit es zu einer guten Luftumwälzung innerhalb der Abscheidekammer kommt, womit auch ein guter Wärmeaustausch verbunden ist. Auf diese Weise wird die unerwünschte Abscheidung grober Kristalle an den Wänden und Austragsvorrichtungen verhindert und ausserdem der Abscheidevorgang wesentlich beschleunigt.

Zur Erzielung des erfindungsgemäßen Effektes ist es bevorzugt, dem Cyanurchloriddampf Kaltluft in Mengen von 50 bis 75 kg pro 100 kg Cyanurchloriddampf zuzumischen. Als besonders vorteilhaft hat sich eine Anordnung von mehreren, insbesondere vier oder mehr Kaltluft-Düsen erwiesen, welche gegenüber der Cyanurchloridzuleitung schräg und tangential zum Cyanurchlorid-Strom angeordnet sind. Diese schnell austretenden Kaltluftströme haben eine Ejektorwirkung und reißen proportional zu ihrer Maße und dem Quadrat ihrer Geschwindigkeit Raumluft mit. Die angesaugte Raumluftmenge beträgt im allgemeinen das $10^3$-fache der eingeblasenen Ejektorluftmenge. Die intensive Durchmischung dieser großen Gasmenge führt in Sekunden zu einem Abschreckeffekt, der das Cyanurchlorid in besonders feinteiliger Form auskondensieren läßt. Das Cyanurchlorid scheidet sich dann sehr rasch im Luftstrom ab und fällt hierbei in einer besonders feinteiligen Form (vorzugsweise besitzen höchstens 3 bis 4% der Teilchen eine Korngröße >60 μm) an. Das Produkt wird im unteren Teil der Abscheidekammer ausgetragen, während die praktisch cyanurchloridfreien Abgase im oberen Teil der Kammer abgezogen werden. Neben dieser Feinteiligkeit besitzt das erfindungsgemäß hergestellte Cyanurchlorid überraschenderweise bereits auch einen sehr hohen Reinheitsgrad (Cyanurchloridgehalt >99,5%, Hydroxytriazine <0,5%, Polytriazine <0,1%) und kann deshalb in den meisten Fällen ohne weitere Reinigungsoperationen direkt weiterverarbeitet werden. Mit dem erfindungsgemäßen Cyanurchlorid wird z.B. Atrazin in einer Ausbeute von 99% erhalten, was mit nach dem Stand der Technik erhaltenem Cyanurchlorid direkt nicht möglich war. Die extrem hohe Kornfeinheit führt außerdem zu einer starken Erhöhung der Reaktivität. Die Reaktionszeit zur Herstellung von Atrazin wird beträchtlich reduziert.

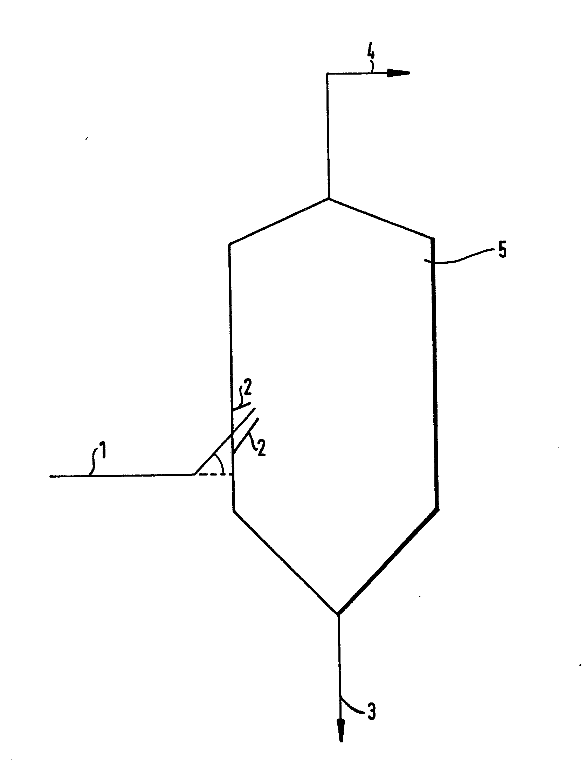

Das erfindungsgemäße Verfahren wird anhand der beigefügten Zeichnung und der Beispiele näher erläutert.

Die Zeichnung zeigt schematisch die erfindungsgemäße Gewinnung von festem Cyanurchlorid aus dem bei der Trimerisierung des Chlorcyans anfallenden Reaktionsgasgemisch. Der Cyanurchloriddampf wird durch Leitung (1) der Abscheidekammer (5) im unteren Teil zugeführt. Die Leitung (1) ist am Eintritt in die Kammer (5) schräg nach oben gerichtet. Durch die Düsen (2) wird Kaltluft eingeleitet. Nach der Desublimation wird der Feststoff über Leitung (3) ausgetragen, während die Abgase über die Leitung (4) am oberen Teil der Abscheidekammer (5) abgezogen werden.

Beispiel 1

Nach dem Austritt aus dem Trimerisierungsreaktor wird der anfallende Cyanurchloriddampf, der noch Verunreinigungen wie z.B. Chlorcyan oder Chlor enthält, in einer Menge von 250 kg/h in den unteren Teil der Abscheidekammer (5) gemäß Zeichnung eingeleitet, wobei die Zuleitung (1) gegenüber der Horizontalen einen Winkel von 45% aufweist. Gleichzeitig leitet man 135 kg/h Kaltluft mit einer Temperatur von 25°C durch mehrere Düsen (2) in die Abscheidekammer (5) und bewirkt damit die Abscheidung des Cyanurchlorids. Während die Kaltluft zusammen mit den Restgasen am oberen Teil der Abscheidekammer abgezogen wird, fällt das feinteilige, feste Cyanurchlorid am Boden der Abscheidekammer an, wo es ausgetragen wird. Es weist folgende Kornverteilung auf:

| | |
|---|---|
| >125 μm | 0% |
| 125–63 μm | 1,6% |
| 40–63 μm | 9,5% |
| <40 μm | 88,9% |

Beispiel 2

Wie im Beispiel 1 beschrieben, werden 250 kg Cyanurchloriddampf stündlich in die Abscheidekammer zusammen mit 135 kg Kaltluft mit einer Temperatur von 10°C eingeleitet und abgeschieden.

Das anfallende Cyanurchlorid besitzt folgende Kornverteilung:

| | |
|---|---|
| >60 μm | 3% |
| 40–60 μm | 30% |
| <40 μm | 67% |

**Patentansprüche**

1. Verfahren zur Gewinnung von festem Cyanurchlorid aus dem bei der Trimerisierung von Chlorcyan anfallenden Cyanurchloriddampf durch Abscheidung in einer Abscheidekammer, dadurch gekennzeichnet, daß man den Cyanurchloriddampf und kalte Luft durch schräg nach oben gerichtete Zuleitungen derart in den unteren Teil einer Abscheidekammer einleitet, daß der Neigungswinkel der Zuleitung gegenüber der Horizontalen 10 bis 80° beträgt, die Kaltluft eine Temperatur von 0 bis 60°C besitzt und man dem Cyanurchlorid die Kaltluft in einer Menge von 20 bis 150 kg pro 100 kg Cyanurchloriddampf zudosiert.

2. Verfahren nach Anspruch 1, dadurch gekennzeichnet, daß der Neigungswinkel der Zuleitung gegenüber der Horizontalen 30 bis 50° beträgt.

3. Verfahren nach Anspruch 1 oder 2, dadurch gekennzeichnet, daß die Kaltluft eine Temperatur von 10 bis 25°C besitzt.

4. Verfahren nach einem der Ansprüche 1 bis 3, dadurch gekennzeichnet, daß man dem Cyanurchlorid Kaltluft in Mengen von 50 bis 75 kg pro 100 kg Cyanurchloriddampf zudosiert.

5. Verfahren nach einem der Ansprüche 1 bis 4, dadurch gekennzeichnet, daß man dem Cyanurchlorid die Kaltluft durch mehrere, tangential zum Cyanurchlorid-Gasstrom und schräg angeordnete Düsen zumischt.

**Claims**

1. Process for the optaining of solid cyanuric chloride from the cyanuric chloride vapour optained in the case of the trimerisation of cyanogen chloride by deposition in a deposition chamber, characterised in that one passes the cyanuric chloride vapour and cold air through obliquely upwardly directed inlet pipes into the lower part of a deposition chamber in such a manner that the angle of inclination of the inlet pipe with regard to the horizontal amounts to 10 to 80°, the cold air possesses a temperature of 0 to 60 °C and one doses the cold air into the cyanuric chloride in an amount of 20 to 150 kg per 100 kg of cyanuric chloride vapour.

2. Process according to claim 1, characterised in that the angle of inclination of the inlet pipe with regard to the horizontal amounts to 30 to 50°.

3. Process according to claim 1 or 2, characterised in that the cold air possesses a temperature of 10 to 25 °C.

4. Process according to one of claims 1 to 3, characterised in that one doses cold air into the cyanuric chloride in amounts of 50 to 75 kg per 100 kg of cyanuric chloride vapour.

5. Process according to one of claims 1 to 4, characterised in that one admixes the cold air with the cyanuric chloride by means of several nozzles arranged tangentially to the cyanuric chloride gas stream and obliquely.

## Revendications

1. Procédé pour l'obtention de chlorure de cyanuryle solide à partir de la vapeur de chlorure de cyanuryle obtenue lors de la trimérisation de chlorocyane, par précipitation dans une chambre de séparation, caractérisé en ce que, via des conduites d'alimentation dirigées en oblique vers le haut, on introduit la vapeur de chlorure de cyanuryle et de l'air froid dans la partie inférieure d'une chambre de séparation de telle sorte que l'angle d'inclinaison de chaque conduite d'alimentation vis-à-vis de l'horizontale atteigne 10 à 80°, que l'air froid ait une température de 0 à 60 °C, tandis que, au chlorure de cyanuryle, on ajoute l'air froid en une quantité de 20 à 150 kg/100 kg de vapeur de chlorure de cyanuryle.

2. Procédé selon la revendication 1, caractérisé en ce que l'angle d'inclinaison de chaque conduite d'alimentation vis-à-vis de l'horizontale est de 30 à 50°.

3. Procédé selon la revendication 1 ou 2, caractérisé en ce que l'air froid a une température de 10 à 25 °C.

4. Procédé selon une des revendications 1 à 3, caractérisé en ce que, au chlorure de cyanuryle, on ajoute de l'air froid en quantités de 50 à 75 kg/100 kg de vapeur de chlorure de cyanuryle.

5. Procédé selon une des revendications 1 à 4, caractérisé en ce que, au chlorure de cyanuryle, on ajoute l'air froid via plusieurs buses disposées tangentiellement au courant du chlorure de cyanuryle gazeux et en oblique.